# EUROPÄISCHE PATENTSCHRIFT

(11) **EP 0 711 132 B1**
(45) Veröffentlichungstag und Bekanntmachung des Hinweises auf die Patenterteilung: **24.03.1999**
(21) Anmeldenummer: 94915501.4
(22) Anmeldetag: 01.06.1994
(51) Int. Cl.: A61B 17/74, A61B 17/80

(54) **GABELPLATTE**
FORKED BONE PLATE
PLAQUE VISSEE BIFURQUEE

(43) Veröffentlichungstag der Anmeldung: 15.05.1996
(73) Patentinhaber: Synthes AG, Chur, 7002 Chur (CH)
(72) Erfinder: BERGER, Roger, CH-4413 Büren (CH); OCHSNER, Peter, E., CH-4402 Frenkendorf (CH); WELTE, Thomas, D-79576 Weil (DE)
(74) Vertreter: Lusuardi, Werther Giovanni, Dr.
(86) Internationale Anmeldenummer: CH9400102
(87) Internationale Veröffentlichungsnummer: WO9532674

(56) Entgegenhaltungen:
- EP-A- 0 009 327
- DE-A- 1 813 807
- DE-A- 2 602 900
- FR-A- 1 487 486
- FR-A- 2 606 268

## Beschreibung

Die Erfindung bezieht sich auf eine Knochenplatte in der Form einer Gabelplatte.

Eine solche Knochenplatte, welche beispielsweise aus der FR-A-2606268 LANDOS bekannt ist, eignet sich allgemein zur Versorgung von Frakturen und für Osteotomien insbesondere am proximalen und distalen Femur, an der proximalen Tibia und am proximalen Humerus oder für Arthrodesen an allen Gelenken. Entsprechend der Vielfalt der Anwendungsmöglichkeiten ist die Anzahl von verschiedenen Ausführungsformen solcher Gabelplatten sehr gross, was zu einer aufwendigen und komplizierten Lagerhaltung und Logistik führt.

Hier will die Erfindung Abhilfe schaffen. Der Erfindung liegt die Aufgabe zugrunde, eine gattungsgemässe Knochenplatte zu schaffen, welche sich ohne grossen Aufwand und auch noch intraoperativ an die verschiedenen Einsatzmöglichkeiten anpassen lässt.

Die Erfindung löst die gestellte Aufgabe mit einer Knochenplatte, welche die Merkmale des Anspruchs 1 aufweist.

Weitere vorteilhafte Ausgestaltungen der Erfindung sind in den abhängigen Ansprüchen gekennzeichnet.

Die gabelartigen Zinken sind - je nach Einsatzgebiet - um einen Winkel α von 60° bis 150° (z.B. 87°, 95° oder 135°) gegenüber der Längsachse des Schaftteils abgewinkelt und individuell verlängerbar. Dies kann im wesentlichen auf zwei verschiedene Arten erfolgen.
Bei einer ersten Ausführungsform sind die Zinken an ihren freien Enden mit Mitteln versehen, welche eine Verlängerung der Zinken durch Verbindung mit einem longitudinalen Verlängerungsteil, z.B. einem in das entsprechend ausgestaltete Zinkenende einschraubbaren Verlängerungsbolzen, gestatten.
Bei einer zweiten Ausführungsform sind die Zinken durchgehend hohl ausgebildet, so dass ein longitudinaler Verlängerungsteil, z.B. eine Knochenschraube, über das freie Ende der hohlen Zinken hinaus in den Knochen schraubbar ist.
Durch die Verlängerbarkeit der Zinken ergibt sich eine stark reduzierte Lagerhaltung (es sind weniger Modelle notwendig; im Prinzip genügt sogar ein einziges universell einsetzbares und adaptierbares Modell).

Bei einer bevorzugten Ausführungsform der Erfindung gabeln sich die Zinken bereits vor dem Ende des Schaftteils auf, und zwar vorzugsweise mindestens um 1 mm vor der Abwinkelung um den Winkel α gegenüber der Längsachse. Der Betrag der vorzeitigen Aufspaltung der Zinken ist abhängig von der Plattenkonstruktion und Dimensionierung und insbesondere von der Dicke des Schaftteils. In Relation zu letzterem sollten die Zinken zweckmässigerweise um mindestens das 0,1-fache, vorzugsweise mindestens um das 0,3-fache der Dicke des Schaftteils vor der Abwinkelungsstelle entspringen.

Die vorzeitige Aufgabelung der Zinken bereits im longitudinalen Schaftteil ergibt eine bessere Stabilität der Platte sowie insgesamt eine verbesserte Bruch- und Biegefestigkeit.

Die optimale Anzahl der Zinken richtet sich nach der Verwendung der Gabelplatte; in der Regel werden zweizinkige Platten bevorzugt. Der freie Abstand zwischen den einzelnen Zinken ist ebenfalls vom Verwendungsgebiet abhängig; vorzugsweise beträgt er mindestens die Hälfte des kleinsten Durchmessers der Zinken. Damit eine Knochenschraube zwischen den beiden Zinken Platz finden kann, sollte der Abstand zweckmässigerweise mindestens 3 mm, vorzugsweise mindestens 5 mm betragen. Die Knochenschraube kann durch eine der oberen Bohrungen des Schaftteils schräg nach oben zwischen die beiden zentralen Zinken gesetzt werden, so dass eine besonders stabile Dreieckskonstruktion resultiert. Zu diesem Zweck sollte mindestens eine, vorzugsweise zwei, den Zinken am nächsten gelegene Bohrungen im schaftteil derart ausgebildet sein, dass sie eine axiale Abwinkelung der einzuführenden Knochenschraube um den Winkel β von 10° - 50° gegenüber der Bohrungsachse gestatten. Das Querschnittsprofil der Zinken kann polygonal, vorzugsweise viereckig, oder auch rund sein. Falls die Zinken um einen Winkel α von etwa 90° abgebogen sind, wird ein quadratisches Querschnittsprofil bevorzugt, abhängig von der örtlich gegebenen Knochenstruktur. Falls die Zinken um einen Winkel α von etwa 130° abgebogen sind, wird ein rundes Querschnittsprofil bevorzugt, abhängig von der örtlich gegebenen Knochenstruktur.

Vorzugsweise weisen die Zinken ein stumpfes verlängerbares Ende auf.

Der freie Abstand zwischen den Zinken kann aber auch grösser gewählt werden, z.B. 12 mm oder 14 mm, so dass dazwischen ein Teil einer Endogelenkprothese, z.B. das Tibia- oder Femur-Teil einer Kniegelenksprothese oder das Femurteil einer Hüftprothese Platz findet.

Das orthogonal zur Längsachse stehende Querschnittsprofil des Schaftteils kann je nach Verwendungsart verschiedenartig ausgebildet sein; vorzugsweise ist es trapezoid ausgebildet, wobei die kürzere Grundlinie des Trapezes zu den Zinken gewandt ist. Damit wird wegen der geringeren Knochenabdeckung die Einheilung des Schaftteils verbessert. Zu diesem Zweck ist es auch möglich die zu den Zinken gewandte, zur Auflage auf den Knochen bestimmte Fläche des Schaftteils derart zu strukturieren, dass eine weiter reduzierte Auflagefläche, vorzugsweise eine punktförmige Auflagefläche entsteht.

Das orthogonal zur Längsachse stehende Querschnittsprofil des Schaftteils kann aber auch schienenförmig ausgebildet sein, um dessen Gleitfähigkeit zu erhöhen, falls die Gabelplatte als Komponente eines dynamisierbaren Kombinationsimplantates eingesetzt wird oder zusammen mit einer Trochanterabstützplatte.

Vorteilhaft ist auch die Ausgestaltung mindestens einer Bohrung des Schaftteils als Kompressionsloch in einer oder zwei Richtungen der Längsachse.
Einzelne oder alle der Bohrungen des Schaftteils können auch konisch ausgebildet sein, wobei die Verjüngung gegen die Zinken hin erfolgt. Diese Ausgestaltung ermöglicht die Verwendung von Knochenschrauben mit entsprechend gestalteten konischen Köpfen, so dass eine rigide Verbindung zwischen Schrauben und Platte resultiert.

Bei einer weiteren Ausführungsform kann die Orthogonale auf der durch die Zinken definierten Ebene einen von 0° abweichenden Winkel ε zur Längsachse des Schaftteils einschliessen. Zu diesem Zweck kann der Schaftteil mit einem Drehgelenk versehen werden, dessen Drehachse senkrecht zum Schaftteil steht und um welches das eine Ende des Schaftteils mit den Zinken gegenüber der Längsachse um den Winkel ε abwinkelbar ist.
Statt des Drehgelenkes können die Zinken auch derart ausgestaltet sein, dass die Orthogonale auf ihrer gemeinsamen Ebene unter einem von 0° abweichenden Winkel ε schief zur Längsachse des Schaftteils verläuft.

Bei einer weiteren Ausführungsform ist die Breite und/oder Dicke des Schaftteils im Zwischenlochbereich kleiner als im Lochbereich, so dass bei der meist notwendigen Anpassung des Schaftteils an die Knochengeometrie, vorzugsweise der Zwischenlochbereich deformiert wird und der Lochbereich unverändert bleibt und keine Komplikationen bei der Einführung der Fixationsschrauben auftreten.

Die durch die Erfindung erreichten Vorteile sind im wesentlichen darin zu sehen, dass die erfindungsgemässe Knochenplatte mit kleiner Kraft und mit geringer Schädigung der Kortikalis mit einer einfachen Technik in den zu versorgenden Knochen eingebracht werden kann und rotationsstabil wirkt.

Die Erfindung und Weiterbildungen der Erfindung werden im folgenden anhand der teilweise schematischen Darstellungen mehrerer Ausführungsbeispiele noch näher erläutert. Es zeigen:
Fig. 1 eine perspektivische Darstellung der erfindungsgemässen Knochenplatte;
Fig. 2 einen Längsschnitt durch die Knochenplatte nach Fig. 1;
Fig. 3 eine perspektivische Darstellung einer Variante der erfindungsgemässen Knochenplatte;
Fig. 4 eine Ansicht einer weiteren Variante der erfindungsgemässen Knochenplatte; und
Fig. 5 einen Längsschnitt durch eine weiteren Variante der erfindungsgemässen Knochenplatte.

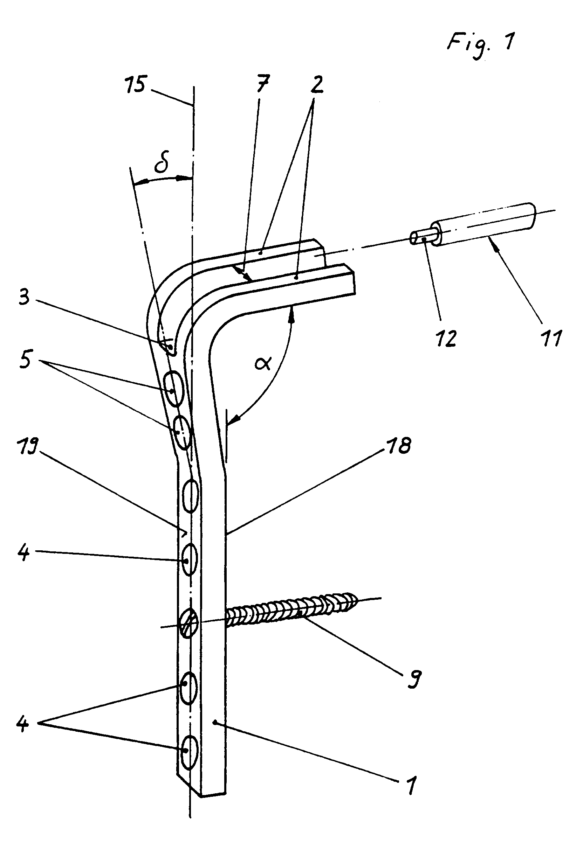

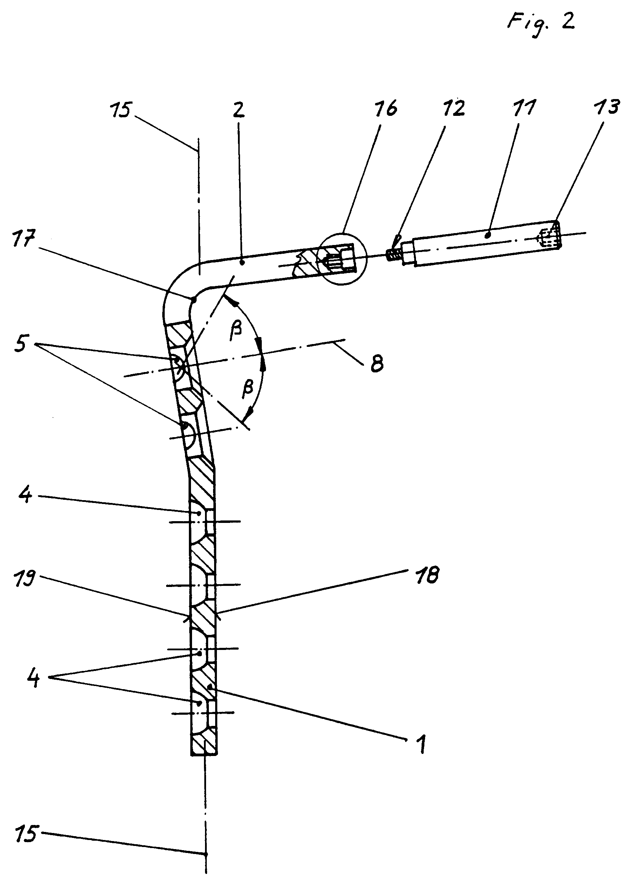

Die in den Fig. 1 und 2 dargestellte Knochenplatte besteht im wesentlichen aus einem longitudinalen Schaftteil 1 an dessen einem Ende sich gabelartig zwei Zinken 2 anschliessen, welche gegenüber der Schaftachse 15 um einen Winkel α von ca. 90° abgebogen sind.

Der Schaftteil 1 hat eine knochenseitige Oberfläche 18 und eine knochenferne Oberfläche 19 welche von einer Anzahl von durchgehenden Bohrungen 4,5 zur Aufnahme von Befestigungsschrauben 9 durchstossen werden. Der obere zinkenseitige Teil des Schaftes ist um den Winkel δ = 15° in Richtung der knochenfernen Oberfläche 19 zurrückgebogen um eine bessere Anpassung an die Anatomie des Femur zu erzielen.

Die beiden, den Zinken 2 am nächsten gelegenen Bohrungen 5 sind - wie in Fig. 2 ersichtlich - derart ausgebildet, dass sie eine beidseitige axiale Abwinkelung der einzufuhrenden Befestigungsschrauben 9 um den Winkel β von bis zu 50° gegenüber der Bohrungsachse 8 gestatten. Zu diesem Zweck erweitern sich die beiden oberen Bohrungen 5 von der Plattenachse 15 aus sowohl zur knochennahen Oberfläche 18 als auch zur knochenfernen Oberfläche 19 in Form eine Hyperboloides.

Die Zinken 2 sind an ihrem freien Ende mit Mitteln 16 in Form eines Hohlgewindes versehen, in welches ein longitudinales Verlängerungsteil 11, mit einem korrespondierenden Gewinde 12 an einem seiner Enden einschraubbar ist. An seinem anderen Ende weist das Verlängerungsteil 11 Eingriffsmittel 13, z.B. in Form eines Innensechskants für einen entsprechenden Schraubenzieher auf, mit dem das Verlängerungsteil 11 in die Zinke 2 eingeschraubt werden kann. Mit einem Satz verschieden langer Verlängerungsteile 11 können die Zinken 2 individuell verlängert werden, je nach Verwendungsart der Gabelplatte.

Die beiden Zinken 2 gabeln sich bereits vor dem Ende des Schaftteils 1 auf und zwar etwa 2 mm vor der Abwinkelungsstelle 17. Bei einer Schaftdicke von 6 mm beträgt dies das 0,33-fache der Schaftdicke. Die beiden Zinken weisen ein quadratisches Querschnittsprofil auf, mit einer Seitenlänge des Quadrates von 6 mm.

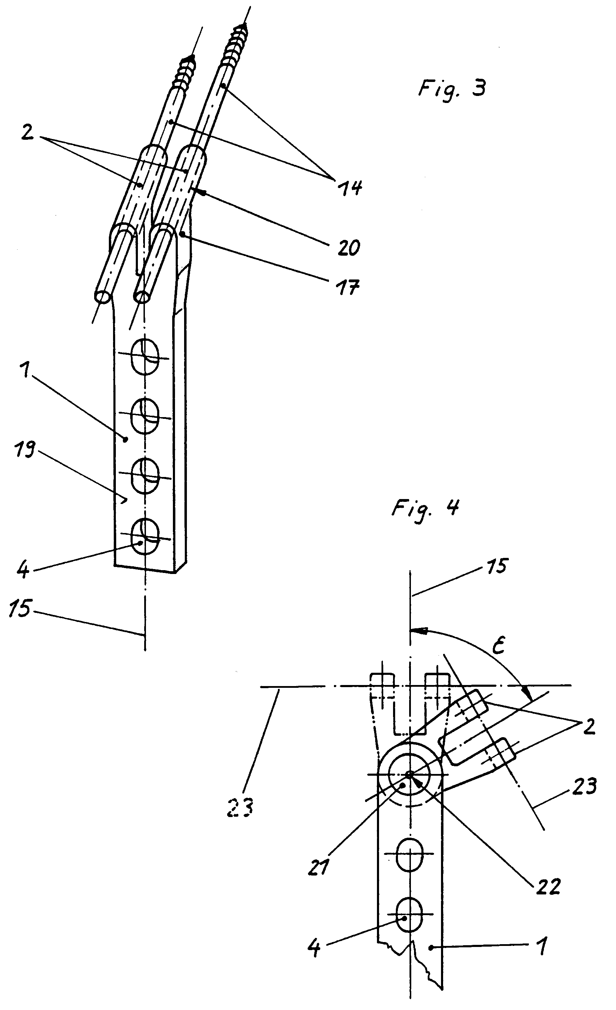

Eine weitere Ausführungsform ist in Fig. 3 dargestellt. Die Verlängerbarkeit der Zinken 2 ist hier dadurch realisiert, dass die Zinken 2 hohl ausgebildet sind, so dass entsprechende Knochenschrauben 14 hindurch geführt werden können. Die durch die Zinken 2 verlaufenden offenen Kanäle 20 beginnen bei der Abwinkelungsstelle 17 und enden am stumpfen Ende der Zinken 2. Die Kanäle 20 können parallel gleitende, komprimierende oder gesperrte Knochenschrauben 14 aufnehmen, welche über das freie Ende der hohlen Zinken 2 hinaus - zu deren Verlängerung - in den Knochen schraubbar sind. Der Schaftteil 1 ist im übrigen analog aufgebaut wie bei der Ausführungsform gemäss den Fig. 1 und 2 mit Ausnahme des Winkel δ der hier 0° beträgt und des Winkels α der hier 135° beträgt.

Fig. 4 zeigt eine weitere Ausführungsform, bei welcher der Schaftteil 1 ein Drehgelenk 21 aufweist, dessen Drehachse 22 senkrecht zum schaftteil 1 steht und um welches das eine Ende des Schaftteils mit den Zinken 2 gegenüber der Längsachse 15 um den Winkel ε abwinkelbar ist. Der Winkel ε wird durch die Orthogonale der Verbindungsebene 23 zwischen den beiden Zinken 2 und der Längsachse 15 des Schaftteiles 1 gebildet.

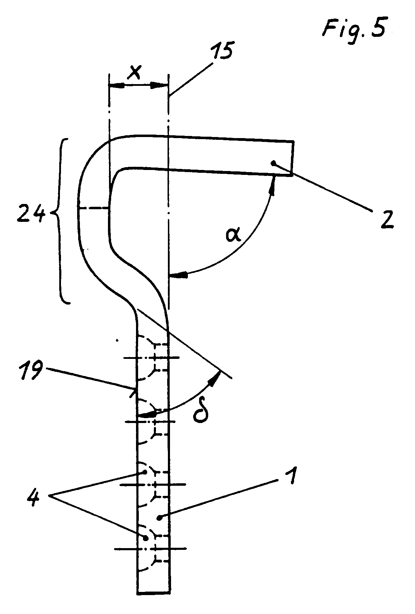

Fig. 5 zeigt eine weitere Ausführungsform, bei welcher der obere zinkenseitige Abschnitt des Schaftteils 1 um den Winkel δ von 5 bis 15° in Richtung der knochenfernen Oberfläche 19 zurückgebogen ist und dann in Form eines Bogenabschnitts 24 (mit dem Mass x) in die Zinken 2 übergeht. Diese Konstruktion ist besonders für Osteotomien am proximalen Femur geeignet.

## Patentansprüche

1. Knochenplatte mit einem longitudinalen Schaftteil (1) und mindestens zwei gabelartig an das eine Ende des Schaftteils (1) angeschlossenen Zinken (2), die unter einem Winkel α von 60° bis 150° gegenüber der Längsachse (15) des Schaftteils (1) abgewinkelt sind wobei die Zinken derart mit Mitteln versehen sind, dass die effektive Zinkenlänge individuell verlängerbar ist, und wobei zwischen den Zinken ein freier Abstand (7) vorhanden ist.

2. Knochenplatte nach Anspruch 1, dadurch gekennzeichnet, dass die Mittel (16) zur effektiven Verlängerung der Zinken (2) an deren freien Enden angeordnet sind, und eine effektive Verlängerung der Zinken (2) durch Verbindung mit einem longitudinalen Verlängerungsteil (11) ermöglichen.

3. Knochenplatte nach Anspruch 2, dadurch gekennzeichnet, dass die Mittel (16) zur Verlängerung der Zinken (2) aus einem Hohlgewinde bestehen, in welches das longitudinale Verlängerungsteil (11), mit einem korrespondierenden Gewinde (12) an einem seiner Enden und vorzugsweise Eingriffsmitteln (13) für einen Eindrehwerkzeug an seinem anderen Ende, einschraubbar ist.

4. Knochenplatte nach Anspruch 1, dadurch gekennzeichnet, dass die die Mittel zur effektiven Verlängerung der Zinken darin bestehen, dass Zinken (2) hohl ausgebildet sind zur Aufnahme von Knochenschrauben (14), die über das freie Ende der hohlen Zinken (2) hinaus zur effektiven Verlängerung der Zinken (2) in den Knochen schraubbar sind.

5. Knochenplatte nach einem der Ansprüche 1 bis 4, dadurch gekennzeichnet, dass die Zinken (2) bereits vor dem einen Ende im Schaftteil (1) entspringen, vorzugsweise mindestens 1 mm vor der Abwinkelungsstelle (17) um den Winkel α gegenüber der Längsachse (15).

6. Knochenplatte nach Anspruch 5, dadurch gekennzeichnet, dass die Zinken (2) mindestens um das 0,1-fache, vorzugsweise mindestens um das 0,3-fache der Dicke des Schaftteils (1) vor der Abwinkelungsstelle (17) entspringen.

7. Knochenplatte nach einem der Ansprüche 1 bis 6, dadurch gekennzeichnet, dass der freie Abstand (7) zwischen den einzelnen Zinken (2) mindestens 3 mm, vorzugsweise mindestens 5 mm beträgt.

8. Knochenplatte nach Anspruch 7, dadurch gekennzeichnet, dass der freie Abstand (7) zwischen den einzelnen Zinken (2) mindestens 12 mm, vorzugsweise mindestens 14 mm beträgt.

9. Knochenplatte nach einem der Ansprüche 1 bis 6, dadurch gekennzeichnet, dass der freie Abstand (7) zwischen den einzelnen Zinken (2) mindestens die Hälfte des kleinsten Durchmessers der Zinken (2) beträgt.

10. Knochenplatte nach einem der Ansprüche 1 bis 9, dadurch gekennzeichnet, dass der Schaftteil (1) mit durchgehenden Bohrungen (4,5) zur Aufnahme von Befestigungsschrauben (9) versehenen ist.

11. Knochenplatte nach einem der Ansprüche 1 bis 10, dadurch gekennzeichnet, dass mindestens eine, vorzugsweise zwei, den Zinken (2) am nächsten gelegene Bohrungen (5) derart ausgebildet sind, dass sie eine beidseitige axiale Abwinkelung der einzuführenden Befestigungsschrauben (9) um den Winkel β von 10° - 50° gegenüber der Bohrungsachse (8) gestatten.

12. Knochenplatte nach einem der Ansprüche 1 bis 11, dadurch gekennzeichnet, dass das orthogonal zur Längsachse (15) stehende Querschnittsprofil des Schaftteils (1) trapezoid ausgebildet ist, wobei dessen kürzere Grundlinie zu den Zinken (2) gewandt ist.

13. Knochenplatte nach einem der Ansprüche 1 bis 12, dadurch gekennzeichnet, dass das orthogonal zur Längsachse (15) stehende Querschnittsprofil des Schaftteils (1) schienenförmig ausgebildet ist.

14. Knochenplatte nach Anspruch 13, dadurch gekennzeichnet, dass sie mit einer damit korrespondierenden Trochanterabstützplatte kombinierbar ist.

15. Knochenplatte nach einem der Ansprüche 1 bis 14, dadurch gekennzeichnet, dass die zu den Zinken (2) gewandte, zur Auflage auf den Knochen bestimmte Fläche des Schaftteils (1) derart strukturiert ist, dass eine reduzierte Auflagefläche, vorzugsweise eine punktförmige Auflagefläche resultiert.

16. Knochenplatte nach einem der Ansprüche 1 bis 15, dadurch gekennzeichnet, dass mindestens eine Bohrung (4) als Kompressionsloch in einer oder zwei Richtungen der Längsachse (15) des Schaftteils ausgebildet ist.

17. Knochenplatte nach einem der Ansprüche 1 bis 16, dadurch gekennzeichnet, dass mindestens eine als Kompressionsloch wirkende Bohrung (4) konisch ausgebildet ist, wobei die Verjüngung gegen die Zinken (2) hin erfolgt.

18. Knochenplatte nach einem der Ansprüche 1 bis 17, dadurch gekennzeichnet, dass sie genau zwei Zinken (2) aufweist.

19. Knochenplatte nach einem der Ansprüche 1 bis 17, dadurch gekennzeichnet, dass sie mindestens drei Zinken (2) aufweist, welche nicht in einer gemeinsamen Ebene liegen.

20. Knochenplatte nach einem der Ansprüche 1 bis 19, dadurch gekennzeichnet, dass die Zinken (2) ein polygonales, vorzugsweise ein quadratisches Querschnittsprofil aufweisen.

21. Knochenplatte nach einem der Ansprüche 1 bis 19, dadurch gekennzeichnet, dass die Zinken (2) ein rundes Querschnittsprofil aufweisen.

22. Knochenplatte nach einem der Ansprüche 1 bis 21, dadurch gekennzeichnet, dass die Mittel zur effektiven Verlängerung der Zinken darin bestehen, dass die Zinken (2) ein stumpfes verlängerbares Ende aufweisen.

23. Knochenplatte nach einem der Ansprüche 1 bis 22, dadurch gekennzeichnet, dass die Orthogonale der durch die Zinken (2) definierten Ebene (23) einen von 0° abweichenden Winkel ε zur Längsachse (15) des Schaftteils (1) einschliesst.

24. Knochenplatte nach Anspruch 23, dadurch gekennzeichnet, dass der Schaftteil (1) ein Drehgelenk (21) aufweist, dessen Drehachse (22) senkrecht zum Schaftteil (1) steht und um welches das eine Ende des Schaftteils (1) mit den Zinken (2) gegenüber der Längsachse (15) um den Winkel ε abwinkelbar ist.

25. Knochenplatte nach einem der Ansprüche 1 bis 24, dadurch gekennzeichnet, dass die Breite des schaftteils (1) im Zwischenlochbereich kleiner ist als im Lochbereich.

26. Knochenplatte nach einem der Ansprüche 1 bis 25, dadurch gekennzeichnet, dass die Dicke des Schaftteils (1) im Zwischenlochbereich kleiner ist als im Lochbereich.

27. Knochenplatte nach einem der Ansprüche 1 bis 26, dadurch gekennzeichnet, dass der obere zinkenseitige Abschnitt des Schaftteils (1) um den Winkel δ von 5 - 15° in Richtung der knochenfernen Oberfläche (19) zurückgebogen ist.

28. Knochenplatte nach einem der Ansprüche 1 bis 27, dadurch gekennzeichnet, dass der obere zinkenseitige Abschnitt des Schaftteils (1) in Form eines Bogenabschnitts (24) gestaltet ist.

29. Knochenplatte nach einem der Ansprüche 1 bis 28, dadurch gekennzeichnet, dass sie mehrstückig ausgebildet ist.

## Claims

1. Bone plate having a longitudinal shaft section (1) and at least two prongs (2) shaped in forked fashion unitary with one end of the shaft section (1), said prongs (2) being bent at an angle of 60° to 150° relative to the longitudinal axis (15) of the shaft section (1) whereby said prongs (2) have means such that the actual length of the prongs (2) is lengthenable individually and whereby said prongs (2) have a free space (7) between each other.

2. Bone plate according to claim 1, characterized in that the means (16) for the actual lengthening of the prongs (2) are arranged at their free ends and an actual lengthening of the prongs (2) is enabled via connection to a longitudinal extension piece (11).

3. Bone plate according to claim 2, characterized in that the means (16) for lengthening the prongs (2) consist of a hollow thread wherein the longitudinal extension piece (11) is apt to be screwed into by means of a corresponding thread (12) at one of its ends and preferably engaging means (13) for a screw driver at its other end.

4. Bone plate according to claim 1, characterized in that the means (16) for the actual lengthening of the prongs (2) consist therein that the prongs (2) are hollowly shaped for the acceptance of bone screws (14) which are screwable into the bone beyond the free ends of the hollow prongs (2) for the actual lengthening of the prongs (2).

5. Bone plate according to one of the claims 1 to 4, characterized in that the prongs (2) begin already before the one end of the shaft section (1), preferably at least 1 mm before the point (17) at which the plate is bent at an angle α with respect to the longitudinal axis (15).

6. Bone plate according to claim 5, characterized in that the prongs (2) begin at a point which is at least 0,1 times, preferably at least 0,3 times the thickness of the shaft section (1) from the point (17) at which the plate is bent.

7. Bone plate according to one of the claims 1 to 6, characterized in that the free space (7) between the individual prongs (2) is at least 3 mm, preferably at least 5 mm.

8. Bone plate according to claim 7, characterized in that the free space (7) between the individual prongs (2) is at least 12 mm, preferably at least 14 mm.

9. Bone plate according to one of the claims 1 to 6, characterized in that the free space (7) between the individual prongs (2) is at least half the smallest diameter of the prongs (2).

10. Bone plate according to one of the claims 1 to 9, characterized in that the shaft section (1) has through boreholes (4;5) for receiving attachment screws (9).

11. Bone plate according to one of the claims 1 to 10, characterized in that at least one, preferably two, of the boreholes (5) adjacent to the prongs (2) are shaped such that they permit a bilateral bending of the attachment screws (9) to be inserted at an angle β of from 10° to 50° relative to the axes (8) of the boreholes (5).

12. Bone plate according to one of the claims 1 to 11, characterized in that the cross section of the shaft section (1), orthogonal to the longitudinal axis (15), has a trapezoidal shape with the shorter baseline facing the prongs (2).

13. Bone plate according to one of the claims 1 to 12, characterized in that the cross section of the shaft section (1), orthogonal to the longitudinal axis (15), has a rail like shape.

14. Bone plate according to claim 13, characterized in that it is apt to be combined with a trochanter support plate corresponding thereto.

15. Bone plate according to one of the claims 1 to 14, characterized in that the surface of the shaft section (1) determined to be bedded on the bone and adjacent to the prongs (2) is structured such that a reduced bedding surface, preferably a pointlike bedding surface results.

16. Bone plate according to one of the claims 1 to 15, characterized in that at least one borehole (4) is configured as compression hole in one or two directions of the longitudinal axis (15) of the shaft section (1).

17. Bone plate according to one of the claims 1 to 16, characterized in that at least one borehole (4) which works as compression hole has a conical shape tapering towards the prongs (2).

18. Bone plate according to one of the claims 1 to 17, characterized in that it provides exactly two prongs (2).

19. Bone plate according to one of the claims 1 to 17, characterized in that it provides at least three prongs (2) that are not arranged in a common plane.

20. Bone plate according to one of the claims 1 to 19, characterized in that the prongs (2) have a polygonal, preferably quadratic cross section.

21. Bone plate according to one of the claims 1 to 19, characterized in that the prongs (2) have a round cross section.

22. Bone plate according to one of the claims 1 to 21, characterized in that the means to the actual lengthening of the prongs (2) consist therein that the prongs (2) have a blunt extendable end.

23. Bone plate according to one of the claims 1 to 22, characterized in that the prongs (2) define a plane (23) and a line orthogonal to said plane forms an angle ε with the longitudinal axis (15) of the shaft section (1) which is other than 0°.

24. Bone plate according to claim 23, characterized in that the shaft section (1) has a pivot joint (21) with an axis perpendicular to the shaft section (1) and around which the end of the shaft section (1) having the prongs (2) can be bent relative to the longitudinal axis (15) with the angle ε.

25. Bone plate according to one of the claims 1 to 24, characterized in that the width of the shaft section (1) is smaller in the region between the holes than in the region containing holes.

26. Bone plate according to one of the claims 1 to 25, characterized in that the thickness of the shaft section (1) is smaller in the region between the holes than in the region containing holes.

27. Bone plate according to one of the claims 1 to 26, characterized in that the part of the shaft section (1) adjacent the prongs (2) is bent back at an angle δ of from 5° to 15° in the direction of the surface (19) facing away from the bone.

28. Bone plate according to one of the claims 1 to 27, characterized in that the part of the shaft section (1) adjacent the prongs (2) is shaped in the form of an arc section.

29. Bone plate according to one of the claims 1 to 28, characterized in that it comprises a plurality of pieces.

## Revendications

1. Plaque pour os présentant une partie longitudinale formant tige (1) et au moins deux dents (2) qui se raccordent en forme de fourche à une extrémité de la partie formant tige (1) et qui sont coudées d'un angle α de 60° à 150° par rapport à l'axe longitudinal (15) de la partie formant tige (1), les dents étant dotées de moyens permettant d'augmenter individuellement la longueur effective des dents, et une distance libre (7) existe entre les dents.

2. Plaque pour os selon la revendication 1, caractérisée en ce que des moyens (16) permettant d'augmenter la longueur effective des dents (2) sont prévus à l'extrémité libre de ces dernières, et permettent d'augmenter la longueur effective des dents (2) par liaison à une pièce de prolongement longitudinal (11).

3. Plaque pour os selon la revendication 2, caractérisée en ce que les moyens (16) pour prolonger les dents (2) sont constitués d'un filet creux dans lequel on peut visser la pièce de prolongement longitudinal (11), qui est dotée d'un filet (12) complémentaire à l'une de ses extrémités et de préférence de moyens d'engagement (13) pour un outil de vissage à son autre extrémité.

4. Plaque pour os selon la revendication 1, caractérisée en ce que les moyens permettant d'augmenter le longueur effective des dents consistent en ce que les dents (2) sont creuses, pour recevoir des vis pour os (14) servant à augmenter le longueur efficace des dents (2) et qui peuvent être vissées dans l'os par l'extrémité libre des dents creuses (2).

5. Plaque pour os selon l'une des revendications 1 à 4, caractérisée en ce que les dents (2) se séparent déjà en avant de l'une des extrémités de la partie formant tige (1), de préférence au moins 1 mm en avant de l'emplacement du coude (17) formant un angle a par rapport à l'axe longitudinal (15).

6. Plaque pour os selon la revendication 5, caractérisée en ce que les dents (2) se séparent à au moins 0,1 fois, de préférence au moins 0,3 fois l'épaisseur de la partie formant tige (1) en avant de l'emplacement du coude (17).

7. Plaque pour os selon l'une des revendications 1 à 6, caractérisée en ce que la distance libre (7) entre les dents (2) individuelles vaut au moins 3 mm, et de préférence au moins 5 mm.

8. Plaque pour os selon la revendication 7, caractérisée en ce que la distance libre (7) entre les dents (2) individuelles vaut au moins 12 mm, et de préférence au moins 14 mm.

9. Plaque pour os selon l'une des revendications 1 à 6, caractérisée en ce que la distance libre (7) entre les dents individuelles (2) vaut au moins la moitié du plus petit diamètre des dents (2).

10. Plaque pour os selon l'une des revendications 1 à 9, caractérisée en ce que la partie formant tige (1) est traversée par des alésages (4, 5) servant à recevoir des vis de fixation (9).

11. Plaque pour os selon l'une des revendications 1 à 10, caractérisée en ce qu'au moins l'un, et de préférence deux des alésages (5) situés le plus près des dents (2) sont configurés de manière à permettre d'insérer des vis de fixation (9) sous une obliquité axiale bilatérale d'un angle β de 10° à 50° par rapport à l'axe (8) de l'alésage.

12. Plaque pour os selon l'une des revendications 1 à 11, caractérisée en ce que le profil d'une section transversale perpendiculaire à l'axe longitudinal (15) de la partie formant tige (1) est en forme de trapèze dont la petite base est dirigée vers les dents (2).

13. Plaque pour os selon l'une des revendications 1 à 12, caractérisée en ce que la section transversale perpendiculaire à l'axe longitudinal (15) de la partie formant tige (1) présente un profil en forme de rail.

14. Plaque pour os selon la revendication 13, caractérisée en ce qu'elle peut être combinée avec une plaque d'appui sur le trochanter qui lui correspond.

15. Plaque pour os selon l'une des revendications 1 à 14, caractérisée en ce que la surface de la partie formant tige (1), qui tournée vers les dents et est destinée à venir se poser sur l'os, est structurée de manière à obtenir une surface de pose réduite, et de préférence une surface de pose ponctuelle.

16. Plaque pour os selon l'une des revendications 1 à 15, caractérisée en ce qu'au moins un alésage (4) est configuré comme trou de compression dans une ou dans les deux directions de l'axe longitudinal (15) de la partie formant tige.

17. Plaque pour os selon l'une des revendications 1 à 16, caractérisée en ce qu'au moins un alésage (4) travaillant comme trou de compression est configuré en forme de co'ne dont le rétrécissement est dirigé vers les dents (2).

18. Plaque pour os selon l'une des revendications 1 à 17, caractérisée en ce qu'elle présente exactement deux dents (2).

19. Plaque pour os selon l'une des revendications 1 à 17, caractérisée en ce qu'elle présente au moins trois dents (2) qui ne sont pas situées dans un plan commun.

20. Plaque pour os selon l'une des revendications 1 à 19, caractérisée en ce que les dents (2) présentent en section transversale un profil polygonal, de préférence un profil carré.

21. Plaque pour os selon l'une des revendications 1 à 19, caractérisée en ce que les dents (2) présentent en section transversale un profil rond.

22. Plaque pour os selon l'une des revendications 1 à 21, caractérisée en ce que les moyens permettant d'augmenter la longueur effective des dents consistent en ce que les dents (2) présentent une extrémité émoussée qui peut être prolongée.

23. Plaque pour os selon l'une des revendications 1 à 22, caractérisée en ce que la normale au plan (23) défini par les dents (2) forme avec l'axe longitudinal (15) de la partie formant tige (1) un angle ε différent de 0°.

24. Plaque pour os selon la revendication 23, caractérisée en ce que la partie pour tige (1) présente une articulation rotative (21) dont l'axe de rotation (22) est perpendiculaire à la partie pour tige (1), et autour duquel l'extrémité de la partie pour tige (1) portant les dents (2) peut être coudée de l'angle ε par rapport à l'axe longitudinal (15).

25. Plaque pour os selon l'une des revendications 1 à 24, caractérisée en ce que la largeur de la partie formant tige (1) est plus petite dans la région intermédiaire entre les trous que dans la région des trous.

26. Plaque pour os selon l'une des revendications 1 à 25, caractérisée en ce que l'épaisseur de la partie formant tige (1) est plus faible dans les régions intermédiaires entre les trous que dans la région des trous.

27. Plaque pour os selon l'une des revendications 1 à 26, caractérisée en ce que la partie supérieure située du côté des dents de la partie formant tige (1) est coudée vers l'arrière d'un angle δ de 5 à 15°en direction de la surface (19) éloignée de l'os.

28. Plaque pour os selon l'une des revendications 1 à 27, caractérisée en ce que la partie supérieure située du côté des dents de la partie formant tige (1) est configurée en forme d'arc de cercle (24).

29. Plaque pour os selon l'une des revendications 1 à 28, caractérisée en ce qu'elle est réalisée en plusieurs pièces.
